# EUROPEAN PATENT APPLICATION

(11) **EP 2 614 830 A1**
(43) Date of publication of application: **17.07.2013**
(21) Application number: 12425005.1
(22) Date of filing: 12.01.2012
(51) Int. Cl.: A61K 36/8962

(54) **Alliacrae hydrolates for use as a medicament, food and aromatic ingredient for foods and food products**

(71) Applicant: Societã Agricola Taflo s.s. di Visentin Lucia & C., 36026 Pojana Maggiore (VI) (IT)
(72) Inventor: Visentin, Lucia, 36026 Pojana Maggiore Vicenza (IT); Negretto, Pierguido, 36026 Pojana Maggiore Vicenza (IT)
(74) Representative: Zambardino, Umberto

(57) **Abstract**

The present invention relates to the use of Alliaceae hydrolates, such as hydrolates of onion, garlic and the like as medicament, particularly for cosmetic and therapeutic treatments or as a flavoring ingredient in food products. Such Alliaceae hydrolates can be used as such in cosmetic or therapeutic treatments, including aromatherapy, or alternatively may constitute an ingredient (active ingredient) for the manufacture of a cosmetic or pharmaceutical product. The above hydrolates can be obtained by distillation techniques of an Alliaceae plant material which are per se conventional, in particular by steam distillation.

## Description

### Field of application

The present invention relates to the use of Alliaceae hydrolates as a medicament, in particular for use in cosmetic and therapeutic treatments.

The present invention also relates to hydrolates of Alliaceae as such for use as a food and the use of said hydrolates as flavouring ingredients in foods or food preparations.

### Prior art

As it is known, the distillation of aromatic plants, usually carried out under a flow of steam, results in the extraction of volatile and odorous substances from said plants obtaining a biphasic product wherein an organic fraction consisting of essential oils and a watery fraction consisting of hydrolates, commonly called also "floral waters" or cohobate are present.

Essential oils consist essentially of water-insoluble aromatic substances coming from the starting plant material whereas hydrolates contain essentially aromatic substances from said plant material which are water-soluble.

Essential oils and hydrolates have different specific weights with the former that are lighter and therefore constitute the supernatant of the biphasic product of the distillation. Therefore, essential oils and hydrolates can be easily separated from each other in an appropriate condenser/ separator wherein condensed vapors from the distillation are collected.

Also for Alliaceae, such as onion, garlic and the like, the steam distillation of the plant material results in obtaining essential oils and hydrolates (floral waters). In that case, the essential oils have specific weight higher than that of water so that they form the lower portion of the biphasic product of the distillation and are thus recovered from the bottom of the separation system.

It is well known that essential oils from Alliaceae have different applications in particular in cosmetic and therapeutic fields, as well as in the food sector. They are widely used in various forms (such as creams, lotions, ointments, etc.) for cosmetic or therapeutic treatments, in particular for aromatherapy while in the food sector they are often used as flavouring ingredients in foods or food preparations such as sauces, gravies, marinades, sausages, soups, savory meats etc..

On the contrary, applications of hydrolates from distillation of Alliaceae are not known to date.

However, essential oils of Alliaceae, like essential oils extracted from other aromatic plants, constitute a concentrated essence that, at least for cosmetic and therapeutic applications, needs to be strongly diluted with a suitable solvent before use, in order to reduce risks of adverse reactions, in particular hypersensitivity reactions, e.g. in the event of topic application.

Due to the strong hydrophobic nature of said essential oils, the solvents mainly used for dilution are constituted by fatty oils and alcohol. However, the choice of the solvent and the percentage of dilution must be done watchfully depending on the intended use and in particular to minimize toxicological and/or allergy risks and/or possible interactions with substances constituting the essential oil which may affect the benefic properties of the latter.

### Summary of the invention

It has now been found surprisingly that hydrolates obtained from distillation of a plant material of Alliaceae, although composed essentially of substances (water-soluble) different from those (fat-soluble) forming the corresponding essential oils of Alliaceae, have beneficial properties comparable to those offered by said essential oils while, at the same time, they offer indubitable advantages compared to the latter in terms of simplicity and safety of use as it will be clearer from the following of the present description.

The hydrolates of Alliaceae therefore lend themselves to be employed for uses similar to the current uses of the corresponding essential oils of Alliaceae both as a substitute for said essential oils, and optionally as a complement to them, while offering greater safety and easy of use.

Therefore, in one aspect, the present invention relates to an hydrolate of Alliaceae for use as a medicament.

In another aspect, the present invention relates to an hydrolate of Alliaceae for use as such in a cosmetic treatment.

In yet another aspect, the present invention relates to an hydrolate of Alliaceae for use as ingredient in the formulation of a cosmetic product.

In a further aspect, the present invention relates to an hydrolate of Alliaceae as such for use as a food.

In a still further aspect, the present invention relates to the use of an hydrolate of Alliaceae as flavouring ingredient for foods or food preparations.

The hydrolate is obtained by distillation, preferably by steam distillation, of a plant material of Alliaceae, according to techniques per se conventional.

The use as a medicament includes preferably the use of hydrolates obtained from the distillation of plant material of Alliaceae in the therapeutic treatments, especially for aromatherapy.

In the cosmetic or therapeutic treatments, said hydrolates may be advantageously used as such, i.e. as obtained in the form of an aqueous solution containing water-soluble aromatic essences of a plant material of Alliaceae after distillation of such a plant material and separation from the corresponding essential oils.

Alternatively, said hydrolates, as obtained from the distillation of plant material of Alliaceae and separation from the respective essential oils, can be diluted with an aqueous medium, preferably water, before use in cosmetic and therapeutic treatments.

Moreover, said hydrolates can be used as ingredients for the formulation of cosmetic or pharmaceutical compositions for use in such treatments.

In any case, it has been observed that the hydrolates resulting from the distillation of Alliaceae have beneficial properties similar to those of the respective essential oils, but compared to the latter, have advantageously a more delicate and balanced action. Therefore, they lend themselves to a wider applicability in the context of the uses covered by the present invention in particular to all those categories of individuals for whom the essential oil may be too "aggressive" and thus lead more easily to undesirable side effects (e.g. allergies). Such categories may include, for example, pregnant women, elderly, children or subjects with specific diseases for which the use of essential oil, for example in cosmetic or therapeutic treatments, may be undesirable, or in special cases even dangerous.

It should also be noted that hydrolates from the distillation of Alliaceae do not require special preparation before their application in the uses provided by the present invention.

They can in fact be used as such in many applications, particularly in therapeutic and cosmetic treatments, or alternatively in a diluted form. Advantageously, the dilution can be performed without the use of organic solvents (particularly alcohol), that is through an aqueous medium, such as an aqueous solution, preferably water. Therefore, the dilution can be done very simply and at a much reduced costs compared to the use of an organic solvent for dilution. Furthermore, the use of water as a solvent for dilution does not introduce any factor/risk of toxicity or allergy that might instead arise from the use of organic solvents such as alcohol, fatty acids and the like.

In addition, there is to say that the dilution of the hydrolates of Alliaceae can also be provided upstream from the manufacturer after the separation of the same from the corresponding essential oils, in order to provide advantageously a product ready for use for each specific need.

### Detailed Description

The term "Alliaceae" means in this description a family of monocotyledonous plants comprising mostly the genus *Allium* and often also classified among the Liliacee. This cosmopolitan family includes herbaceous plants most often fitted with bulb, sometimes rhizome. The symmetrical flowers have superior ovary and form an umbrella-like inflorescence and the fruit is like a capsule.

Alliaceae plants usable in the present invention include as a not-limiting example: onion, leek, garlic, scallion, borettane and chive.

In the present invention, Alliaceae plants particularly preferred are onion and borettane.

The term "plant material of Alliaceae" means in this description any material of plant nature consisting of or comprising plants of at least one type belonging to the family of Alliaceae (e.g. plants of onion and/or garlic), said material comprising those plants either as a whole (intact) and/or parts of such plants. The Alliaceae plants can be processed in part beforehand immediately after harvest and in part stored in a controlled environment until use.

Preferably, the plants of Alliaceae are subjected to a pre-processing in order to isolate the parts of interest to be subjected to distillation. Those parts may include, for example, the fruit, bulb, rhizome, flower and/or leaves.

In addition, these plants or parts of them may also be subjected, prior to distillation, to one or more mechanical treatments, in particular and preferably to get the maximum performance during the extraction process of the aromatic essences by means of said distillation. Such mechanical treatments include without limitation washing, optional peeling and chopping (grinding). The grinding is carried out in order to minimize the Alliacea to be distilled as much as possible so as to expose more surface of the plant as possible to the action of the steam.

In the present invention, the distillation can be effected by any technique per se conventional employing an aqueous extraction medium or in the absence of the aqueous medium (i.e. dry extraction). Such techniques include, without limitation distillation with direct or indirect steam, hydro-diffusion and extraction with CO2.

Particularly preferred is the distillation with direct or indirect steam.

The distillation is carried out using appropriate apparatuses in itself conventional. For example, in the case of steam distillation, the apparatus may include a receptacle (distiller) tightly closed and in fluid communication with a condenser and a steam generator through respective openings for steam inlet and for distillate outlet (steam/volatile oils mixture).

The steam can be introduced directly into the distillation chamber containing the plant material, especially below it, so that the plant material is crossed by water vapor. In this case the distillation is carried out in a flow of direct steam. Alternatively, the distiller may be equipped with a heating jacket outside the distillation chamber wherein the water vapor is introduced. In this case, the plant material is placed in the distillation chamber possibly together with a predetermined amount of water and the mixture is heated indirectly by the steam crossing said outer jacket.

The apparatus may also include a condenser and a separator in fluid communication with each other for condensation and separation of the distillate.

Preferably, the plant material intact or previously processed for obtaining the parts of the plant of interest for the extraction, as the case may be, is finely chopped for maximum efficiency in the process of extraction and is placed in the distiller together with a prefixed amount of water. Then, the plant material and the added water are heated by a flow of indirect water vapor under pressure (coming from the generator) to a preset temperature for a extraction time also preset. The steam generated by heating the plant mass and the added water carries volatile oils contained in the plant itself in the condenser and the resulting steam/volatile oils mixture is condensed and then conveyed into the collection vessel wherein it separates into essential oil and hydrolate due to the different specific weight.

Preferably, the steam has a temperature between 100 °C and 130 °C and the distillation time is between 120 minutes and 180 minutes. This time is usually enough for most of the essential oil contained in plant material subjected to distillation to be extracted by the steam directly or indirectly.

At the end of the distillation, the supply of steam is suspended and the essential oil passed through the condenser is collected in the separator and finally separated from the hydrolate which has conveyed it during the distillation.

In the case of distillation of plant material of Alliaceae, the hydrolate resulting from this distillation has a specific weight less than that of the corresponding essential oil so that it separates from the top of the separation vessel. The precipitation / separation of the essential oil is substantially contemporaneous with the distillation.

Finally, the hydrolate is recovered from the separator for example allowing it to exit from a specific outlet opening located at the top of the separator to predetermined height. Similarly, the essential oil can be recovered from the bottom of the separator through a specific outlet opening located for example near the bottom of the separator.

The hydrolate recovered can be packaged as such in suitable containers, which may be rigid or flexible according to preset doses. Such containers can be made with a material suitable for food at least on the inner side. Examples of suitable containers include as a not-limiting example bottles and similar containers, containers made of aluminum having inner lining suitable for food.

The container (or package) may exhibit a note on the outside (for example in the form of a label) indicating other than the type of product, the intended use for specific treatments such as cosmetic or therapeutic treatments or in the food industry.

In some cases, it may be appropriate that such hydrolate is diluted prior to be employed for the intended therapeutic/cosmetic use or use for foods.

In such cases, the dilution can be performed prior to packaging (i.e. directly from the manufacturer) with the advantage of providing a product ready for consumption directly to the specific needs of use of a diluted hydrolate. Alternatively, the hydrolate can be diluted, if necessary, by an end user or by specialized personnel as in the case of performing cosmetic or therapeutic treatments or, as in the food sector, directly in industrial processes for the preparation of food products whose formulation involves the addition of such a diluted hydrolate, for example as a flavoring agent.

The dilution, where provided, is preferably carried out with an aqueous medium, particularly water, according to predetermined ratios depending on the intended use.

Preferably, the dilution is carried out according to a ratio between volume of hydrolate as recovered from the distillation and volume of the aqueous medium of between 1:1 and 1:1000.

The Alliaceae hydrolates described above, as obtained and recovered by distillation or after subsequent dilution with an aqueous medium can be used as the sole ingredient in the manufacture of a medicament or a cosmetic for external or internal applications.

In such applications, the Alliaceae hydrolates described above result to be particularly advantageous for their gentle and effective action without side effects. Moreover, since in the above hydrolates the aromatic essence is contained in aqueous solution, the absorption capacity of these aromatic essences and the use of the same by the body is much better.

In particular, the Alliaceae hydrolates have resulted to be topic agents highly effective and soothing, and therefore they are ideal for external applications especially where the drying and irritating properties of alcohol typically contained in conventional drugs or cosmetics (e.g. based on essential oils) are undesirable and/or would advise against the use of these conventional products.

For example, Alliaceae hydrolates can be effectively used as such or in diluted form with water as a lotion for cuts, scrapes and rashes, especially in children and infants, and as a cream for dry, sensitive and inflamed skins.

The above hydrolates may be used as such (possibly after dilution with an aqueous medium) for aromatherapy. The aromatherapy using the above hydrolates can be carried out in various ways for example by topical treatment (massages, fomentations etc.) inhalation (in particular using inhalers or sprays (aerosols), per-mucosal application (for example rinses or gargles and mouthwashes) , olfactory or oral application.

In this aromatherapy, the above hydrolates may achieve benefic effects of both cosmetic type, such as skin toning, and therapeutic type in particular for the treatment of inflammatory diseases (e.g. against bronchitis, throat, rheumatisms), skin or mucous infections, gastrointestinal tract infections, diseases of the genitourinary apparatus exerting antiseptic action, treatment of skin diseases (e.g. dermatitis, rashes, etc.), stimulation of liver functionality.

The above hydrolates may also be used as ingredients for the formulation of cosmetic products or pharmaceutical compositions for use in particular in the treatments specified above. In such cosmetic products and / or pharmaceutical compositions, the above hydrolates may be the sole active substance or may be associated with one or more other active substances as needed. In addition, these cosmetic products and / or pharmaceutical compositions may comprise one or more among carriers and excipients pharmaceutically or cosmetically acceptable which are chosen according to the normal knowledge of persons skilled in these areas and according to specific needs. In this regard, there is to say that the hydrolates of Alliaceae have greater compatibility than alcohol with many of the ingredients normally used for manufacturing the above products or compositions so that these products or compositions can be advantageously manufactured without the use of alcohol. For example, the hydrolates of Alliaceae have a good compatibility for the "bases" commonly used in the formulation of creams and tinctures for medical or cosmetic use so that they can be mixed effectively with these bases thereby avoiding the use of alcohol and its negative typical effects resulting from such use as dryness and irritation of the skin and destabilization of the cream or tincture by separation of the liquid phase.

The cosmetic products and pharmaceutical compositions described above can be in any form of normal use such as lotions, tinctures, ointments, creams, muds, pills, tablets, etc. incorporating the hydrolates of Alliaceae described above.

Cosmetic products incorporating hydrolates of Alliaceae as described above include as a not-limiting example: body lotions, fomentations for face, toners, shampoos, hair dyes, soaps, toothpastes, perfumes, fixing gels etc..

Pharmaceutical compositions incorporating hydrolates of Alliaceae as described above can be used for- the treatment of various diseases including respiratory, digestive, circulatory, reproductive, muscular and nervous system diseases as well as dietary supplements and vitamins.

With regard to the food sector, there is to say that hydrolates of Alliaeceae as above have high organoleptic properties and in particular a more fresh and delicate aroma compared to the corresponding essential oils. This makes the hydrolates of Alliaeceae described above ideal for use as flavoring ingredients in food or food preparations with the possibility, thanks to the ease of use of such hydrolates, to customize the organoleptic properties of the final product as required.

Food products wherein the introduction of the hydrolates of Alliaceae described above may be provided include as a not limiting example: soups, salad dressings, marinades, sauces, food supplements, beverages and spirits, compositions for baked goods.

Finally, it is pointed out that the hydrolates of Alliaceae described above have a more stable quality over time compared to the corresponding essential oils due to the fact that they do not have any additives such as oils or supporting alcohol and even they do not require the addition of antioxidants to increase the shelf life. This aspect is particularly advantageous because it allows preserving the benefic essence of the aromatic plant contained in such hydrolates in an almost intact manner for a very long time. However, it may be appropriate to protect the essence contained in those hydrolates of Alliaceae or the products containing such hydrolates from the exposure to natural light in order to reduce the possibility of degradation phenomena catalyzed by light. This can be achieved for example by packaging the hydrolates as above in dark-glass containers that can act as a filter for ultraviolet rays or in aluminum containers provided with an internal lining suitable to store food.

The present invention will now be described with some examples of realization which are provided for information and not limiting purposes.

### Example 1

### Preparation of an onion hydrolate.

6000 kg of fresh onions (fruit) previously chopped are placed in a distiller equipped internally with a distillation chamber and externally with a heating jacket. In particular, the plant material (fresh chopped onions) is placed inside the distillation chamber and 1200 kg of distilled equal to 20% by weight on the weight of plant material (fresh chopped onions) are added. The distiller is closed hermetically and a stream of indirect water vapor under pressure (2.7 bar) having a flow rate of 700 kg / h and a temperature of 130 °C is introduced into the heating jacket. The steam supply is made for an extraction time of 180 minutes, after which such supply is interrupted.

The mixture of water vapor / essential oils extracted from the plant material exiting from the distiller is condensed in a heat exchanger (condenser) equipped with a cooling jacket and crossed by water at a temperature of 34-35°C and then directed to a collection vessel.

In this way, a liquid two-phase product consisting of essential oil and hydrolate (supernatant) is obtained.

The hydrolate and the essential oil are separated from each other by allowing the essential oil to exit from the bottom of the separator and the hydrolate to exit from the upper part of the same. At the end of the separation 30 liters of hydrolate are obtained which are packaged with preset doses in dark-glass or aluminum bottles using a volumetric measuring device.

The hydrolate obtained as above is particularly useful for health-herbal applications such as lotions, tinctures, ointments, creams, muds, pills, tablets, etc.. and as a flavoring ingredient in the formulation of food products such as soups, sauces, gravies, marinades, concentrates, stocks, etc.

### Example 2

### Preparation of a diluted onion hydrolate

An onion hydrolate is prepared through the distillation and separation process described in Example 1.

This hydrolate is diluted with water at a ratio between volume of hydrolate and volume of water of 1:10.

The diluted hydrolate thus obtained is packaged with preset doses in dark-glass or aluminum bottles using a volumetric dosing.

The diluted hydrolate obtained as above can be used to flavor fresh vegetables such as salads.

## Claims

1. Hydrolate of Alliaceae for use as a medicament.

2. Hydrolate of Alliaceae for use as such in aromatherapy or in the manufacture of a medicament for aromatherapy.

3. Hydrolate of Alliaceae for use as such or in the manufacture of a medicament for the treatment of one or more diseases selected from the group comprising respiratory diseases, digestive diseases, circulatory diseases, reproductive disorders, muscular disorders, nervous system disorders and inflammatory diseases.

4. Hydrolate of Alliaceae for use in the manufacture of a dietary or vitamin supplement.

5. Hydrolat of Alliaceae for use as such in a cosmetic treatment or as an ingredient for the formulation of a cosmetic product.

6. Hydrolate of Alliaceae for use as such as food.

7. Use of an hydrolate of Alliaceae as flavoring ingredient for foods or food preparations.

8. Hydrolate of Alliaceae according to anyone of the preceding claims wherein the hydrolate is obtained from the distillation of a plant material of Alliaceae, said distillation being carried out preferably under a current of steam.

9. Hydrolate of Alliaceae according to claim 8, wherein the hydrolate obtained from the distillation of said plant material of Alliaceae is diluted with an aqueous medium, preferably water.

10. Hydrolate of Alliaceae according to claim 9, wherein said dilution is carried out according to a ratio between volume of hydrolate recovered from said distillation and volume of the aqueous medium of between 1:1 and 1: 1000.

11. Hydrolate of Alliaceae according to anyone of the preceding claims wherein said Alliaceae are selected from the group comprising onion, leek, garlic, scallion, borettane and chive, preferably onion and borettane.

12. Cosmetic product, food or pharmaceutical composition comprising an hydrolate of Alliaceae as defined in anyone of the preceding claims.
